# EUROPEAN PATENT APPLICATION

(11) **EP 4 670 731 A1**
(43) Date of publication of application: **31.12.2025**
(21) Application number: 24184787.0
(22) Date of filing: 26.06.2024
(51) Int. Cl.: A61K 38/17, A61P 9/10, A61P 31/00, A61P 37/02

(54) **HSP70 AND ACTIVE PARTS THEREOF FOR USE IN THE TREATMENT OF IMMUNOPARALYSIS**

(71) Applicant: SurvivX B.V., 6525 EC Nijmegen (NL)
(72) Inventor: DE BEST, Cornelia Maria, 6525 EC Nijmegen (NL); VAN LEEUWEN, Remko, 6525 EC Nijmegen (NL); PICKKERS, Roelof Peter, 6525 GA Nijmegen (NL); KOX, Matthijs, 6525 GA Nijmegen (NL)
(74) Representative: V.O.

(57) **Abstract**

The invention relates to Heat Shock Protein 70 (Hsp70) or a therapeutically active part thereof for use in the treatment or prevention of immune suppression, in particular immunoparalysis.

## Description

### Field of the invention

The invention relates to the field of therapeutic molecules and methods for treating immunosuppression and immunoparalysis resulting from severe disease and disorders. More specifically, the present disclosure relates to Hsp70 and derived peptides for such immunosuppression and immunoparalysis.

### Background of the invention

Proper immunologic balance between pro- and anti-inflammatory forces is necessary for recovery from critical illness. Persistence of a marked compensatory anti-inflammatory innate immune response is termed immunoparalysis. In this state, the severely underactive immune system is unable to detect pathogens, mount an inflammatory response, destroy microbial invaders, or repair damaged tissue places the patient at risk for death from secondary infection and persistent organ failure.

Human leucocyte antigen (HLA)-DR, expressed on antigen-presenting cells, acts as an indicator of immune function. Research advances have highlighted the predictive values of monocytic HLA-DR (mHLA-DR) expression for severity of a variety of diseases. While the regulatory mechanism of altered mHLA-DR expression remains unclear, HLA-DR-/low monocytic myeloid-derived suppressor cells are potent drivers of immunosuppression and poor outcomes in these diseases (Liu et al. 2023).

In a situation of immunoparalysis, the antigen presenting cells express a strongly reduced amount of antigen presenting proteins (HLA-DR) on the cell surface, and the number of antigen presenting cells may also be reduced, which will decrease the effectiveness of the immune system to fight infections. Immunoparalysis can occur after a previous episode of strong (hyper) inflammation due to multiple causes but may also be caused by sources of external origin e.g. immunosuppressing therapies.

If the state of immunosuppression is prolonged and severe, this state has been termed immunoparalysis. A massive proinflammatory response without proper controls is pathologic and places the patient at risk of organ dysfunction and death. Characterized by markedly impaired innate immune function, immunoparalysis is now recognized as a predictor of morbidity and mortality for both children and adults.

The state of immunoparalysis can occur in patients who suffer from bacterial infections, viral infections, sepsis, septic shock, MODS, (severe) injury, severe physical and psychological stress, acute ischemic stroke, spinal cord injury, liver ischemia-reperfusion injury, myocardial infarction, cystic fibrosis, acute coronary syndrome (ACS), pancreatitis, long term hospital and/or ICU admissions, and patients who receive major surgical procedures, cardiopulmonary bypasses, coronary artery bypass grafting (CABG), (immune) oncology therapies, chemotherapies, transplants, immunosuppressing therapies. Moreover in cancer, studies have shown that innate immune cells, such as macrophages, myeloid-derived suppressor cells and neutrophils are highly plastic and may differentiate into immunosuppressive cells depending on signals received in their microenvironment, while NK cells appear to be functionally impaired. It is clear that the development of cancer immunotherapies is impaired by immunoparalysis, and that effective immunotherapy for hematologic tumors is hampered by immune evasion and immune paralysis.

Immunoparalysis could have detrimental implications for the patient, and restoring immune homeostasis is therefore vital to prevent adverse patient outcomes. It has been suggested that immunoparalysis can be reversed by immunomodulatory therapies. Moreover, these immunomodulatory therapies can induce trained immunity, e.g. a functional state of the innate immune response which is characterized by epigenetic reprogramming of innate immune cells. Training of innate immune cells enhances immune responses against microbial pathogens after restimulation. Therapeutic agents evaluated so far (such as GM-CSF or IFN-gamma) are suboptimal. Hence, there remains a need for effective agents to counteract immunoparalysis and/or induce trained immunity.

### Summary of the invention

It is an object of the present invention to provide a novel, effective strategy for counteracting or reversing a suppressed state of the immune state, in particular of immunoparalysis. It is a further object of the invention to provide such strategy to improve quality of life, improve survival, reduce (future) infections, reduce complications of such infections and improve efficacy of therapies, such as cancer therapies.

The invention therefore provides a compound comprising Heat Shock Protein 70 (Hsp70) or a therapeutically active part thereof for use in the treatment or prevention of immune suppression in an individual.

In a further aspect, the invention provides Hsp70 or a therapeutically active part thereof for use in the treatment or prevention of immune suppression in an individual. The immune suppression is preferably immunoparalysis.

In a further aspect, the invention provides a method for treating or preventing immunoparalysis in an individual in need thereof, comprising administering to the individual a therapeutically effective amount of a compound comprising Hsp70 or a therapeutically active part thereof. The immune suppression is preferably immunoparalysis.

In a further aspect, the invention provides a compound comprising Hsp70 or a therapeutically active part thereof for use in reversing immune suppression or inducing trained immunity in an individual.

In a further aspect, the invention provides Hsp70 or a therapeutically active part thereof for use in reversing immune suppression, preferably immunoparalysis, or inducing trained immunity in an individual.

In a further aspect, the invention provides a method for reversing immune suppression, preferably immunoparalysis, in an individual in need thereof, comprising administering to the individual a therapeutically effective amount of a compound comprising Hsp70 or a therapeutically active part thereof.

In a further aspect, the invention provides a compound comprising Hsp70 or a therapeutically active part thereof for use in preventing or counteracting post-trauma infection, such as post-injury infection, secondary infection, and post-surgery infection or complications of such post-trauma infection in an individual.

In a further aspect, the invention provides Hsp70 or a therapeutically active part thereof for use in preventing or counteracting post-trauma infection, such as post-injury infection, secondary infection, and post-surgery infection or complications of such post-trauma infection in an individual.

In a further aspect, the invention provides a method for preventing or counteracting post-trauma infection, such as post-injury infection, secondary infection, and post-surgery infection or complications of such post-trauma infection in an individual in need thereof, preferably by counteracting immunoparalysis, comprising administering to the individual a therapeutically effective amount of a compound comprising Hsp70 or a therapeutically active part thereof.

In a further aspect, the invention provides a compound comprising Hsp70 or a therapeutically active part thereof for use in treating trauma and/or complications thereof, wherein the trauma is selected from the group consisting of critical illness, chronic psychological stress, (severe) physical injury, stroke, such as acute ischemic stroke, spinal cord injury, liver ischemia-reperfusion injury, myocardial infarction, cystic fibrosis, acute coronary syndrome (ACS), pancreatitis, and/or cancer, and/or results from surgery, transplantation, anticancer therapy, immunosuppressing therapy, chemotherapy, long term hospitalisation and/or Intensive Care Unit (ICU) admission.

In a further aspect, the invention provides Hsp70 or a therapeutically active part thereof for use in treating trauma and/or complications thereof in an individual, wherein the trauma is selected from the group consisting of critical illness, chronic psychological stress, (severe) physical injury, stroke, such as acute ischemic stroke, spinal cord injury, liver ischemia-reperfusion injury, myocardial infarction, cystic fibrosis, acute coronary syndrome (ACS), pancreatitis, and/or cancer, and/or results from surgery, transplantation, anticancer therapy, immunosuppressing therapy, chemotherapy, long term hospitalisation and/or Intensive Care Unit (ICU) admission, preferably characterized in that the individual is suffering from immunoparalysis.

In a further aspect, the invention provides a method for treating trauma and/or complications thereof in an individual in need thereof, wherein the trauma is selected from the group consisting of critical illness, chronic psychological stress, (severe) physical injury, stroke, such as acute ischemic stroke, spinal cord injury, liver ischemia-reperfusion injury, myocardial infarction, cystic fibrosis, acute coronary syndrome (ACS), pancreatitis, and/or cancer, and/or results from surgery, transplantation, anticancer therapy, immunosuppressing therapy, chemotherapy, long term hospitalisation and/or Intensive Care Unit (ICU) admission, preferably characterized in that the individual is suffering from immunoparalysis, comprising administering to the individual a therapeutically effective amount of a compound comprising Hsp70 or a therapeutically active part thereof.

In a further aspect, the invention provides a compound comprising Hsp70 or a therapeutically active part thereof for use in improving the efficacy of anticancer therapy in an individual.

In a further aspect, the invention provides Hsp70 or a therapeutically active part thereof for use in improving the efficacy of anticancer therapy in an individual.

In a further aspect, the invention provides a method for improving the efficacy of anticancer therapy in an individual comprising administering to the individual a therapeutically effective amount of a compound comprising Hsp70 or a therapeutically active part thereof.

The Hsp70 or therapeutically active part thereof may comprise modifications, in particular N-terminal, C-terminal modifications and/or internal modifications, such as C-terminal amidation, N-terminal acylation, fatty acid modifications, esterification and combinations thereof. Internal modifications include the presence of non-naturally occurring amino acids and D amino acids, wherein the non-naturally occurring amino acids and D amino acids are correspond the original amino acid.

The Hsp70 or therapeutically active part thereof may be comprised in a compound. Said compound may comprise a further moiety, such as a label or a targeting moiety, or said compound comprises the Hsp70 or therapeutically active part thereof coupled to or encapsulated into a carrier.

The Hsp70 or therapeutically active part thereof may be in the form of a pharmaceutically acceptable salt.

### Detailed description

As used herein, "to comprise" and its conjugations is used in its non-limiting sense to mean that items following the word are included, but items not specifically mentioned are not excluded. In addition the verb "to consist" may be replaced by "to consist essentially of" meaning that a compound or adjunct compound as defined herein may comprise additional component(s) than the ones specifically identified, said additional component(s) not altering the unique characteristic of the invention.

The articles "a" and "an" are used herein to refer to one or to more than one (i.e., to at least one) of the grammatical object of the article. By way of example, "an element" means one element or more than one element.

The word "approximately" or "about" when used in association with a numerical value (e.g. approximately 10, about 10) preferably means that the value may be the given value (e.g. 10), plus or minus 5% of the value (e.g. 10, plus or minus 5%), preferably plus or minus 1% of the value.

The use of the alternative (e.g., "or") should be understood to mean either one, both, or any combination thereof of the alternatives.

The terms "protein" and "polypeptide" refer to compounds comprising amino acids joined via peptide bonds and are used interchangeably. A protein encoded by a gene is not limited to the amino acid sequence encoded by a gene, but may include post-translational modifications of the protein.

In amino acid sequences or variants thereof as defined herein amino acids are denoted by single-letter symbols. These single-letter symbols and three-letter symbols are well known to the person skilled in the art and have the following meaning: A (Ala) is alanine, C (Cys) is cysteine, D (Asp) is aspartic acid, E (Glu) is glutamic acid, F (Phe) is phenylalanine, G (Gly) is glycine, H (His) is histidine, I (Ile) is isoleucine, K (Lys) is lysine, L (Leu) is leucine, M (Met) is methionine, N (Asn) is asparagine, P (Pro) is proline, Q (Gln) is glutamine, R (Arg) is arginine, S (Ser) is serine, T (Thr) is threonine, V (Val) is valine, W (Trp) is tryptophan, Y (Tyr) is tyrosine.

The percentage of identity of an amino acid sequence , or the term "% sequence identity", is defined herein as the percentage of residues of the full length of an amino acid sequence that is identical with the residues in a reference amino acid sequence after aligning the two sequences to achieve the maximum percent identity. E.g. a full length Hsp70 amino acid sequence is compared with a full length Hsp70 reference amino acid sequence and a part of the Hsp70 amino acid sequence is compared with the corresponding part in a Hsp70 reference amino acid sequence. Methods and computer programs for alignment are well known in the art, for example "Align 2". Programs for determining amino acid sequence identity are also well known in the art, for example, the BESTFIT, BLAST and FASTA programs. These programs are readily utilized with the default parameters recommended by the manufacturer.

As used herein, the term "individual" encompasses humans and animals, preferably mammals. Preferably, an individual is a mammal, more preferably a human.

The term "treatment" refers to inhibiting a disease, disorder or condition, i.e., halting or reducing its development or at least one clinical symptom of the disease, disorder or condition, and/or to relieving symptoms of the disease, disorder or condition. In some embodiments, treatment may be administered after one or more symptoms have developed. In other embodiments, treatment may be administered in the absence of symptoms. In some embodiments, treatment may be administered after one or more biomarkers for the disease, disorder or condition is detected. For example, treatment may be administered to a susceptible individual prior to the onset of symptoms. Treatment may also be continued after symptoms have resolved, for example to prevent or delay their recurrence.

As used herein, the term "prevention" refers to precluding or delaying the onset of a disease, disorder or condition and/or the appearance of clinical symptoms of the disease, disorder or condition in a individual that does not yet experience clinical symptoms of the disease, disorder or condition.

The term "therapeutically effective amount," as used herein, refers to an amount of an agent being administered sufficient to relieve one or more of the symptoms of the disease, disorder or condition being treated to some extent. This can be a reduction or alleviation of symptoms, reduction or alleviation of causes of the disease, disorder or condition or any other desired therapeutic effect.

The results included herein demonstrate a significant reversal of immunoparalysis after exposing endotoxin-induced immune suppressed monocytes to Hsp70 variants, which is a recognized model for immunoparalysis. In particular, incubating LPS-tolerant monocytes with human or filarial Hsp70 proteins in the presence or absence of LPS significantly enhanced the induction of the proinflammatory cytokines IL-6 and TNF compared to incubation with LPS alone. The reversal effects of the Hsp70 proteins were much stronger than those of IFN-y, a well-known reversal agent. Depending on the dose of the Hsp70 variant, complete reversal of immunoparalysis was possible.

It is known that while IFN-y enhances innate immunity, is also suppresses the adaptive immune responses via upregulation of PD-L1. This finding may provide an explanation as to why IFN-y has been very successful in reversing innate immune defects, such as endotoxin tolerance and impaired *ex vivo* TNF-α production (Chen and Ivashiv 2010; Leentjens et al. 2012, Bundschuh et al. 1997, Döcke et al. 1997), but not in enhancing host competence to fight off (secondary) infections in clinically more relevant models of live bacterial sepsis (Miles et al. 1994, Murphey et al 2004, Murphey and Sherwood 2006). It is hypothesized that Hsp70 and variants do not upregulate PD-L1, which provides it with advantages over IFN-y. In addition, combination immunotherapy with Hsp70 proteins and variants may represent an interesting option to reverse both innate and adaptive immune defects.

In the examples herein, comparable immunological responses in human primary immune cells were induced by filarial (*Setaria digitata*) and human Hsp70 proteins and their C-terminal parts. Both proteins showed comparable abilities in inducing cytokine production and reversing immunoparalysis. Therefore, Hsp70 proteins and variants can be used to treat or prevent immunoparalysis and its consequences.

The proteins and peptides of the invention are effective in combating a suppressed or paralyzed state of the immune system and are therefore capable of improving quality of life, improving survival, reducing (future) infections, reducing of complications of infections, improving efficacy of (cancer) therapies, through immunomodulation by neutralizing the suppressed or immune state, or reactivating and restoring the immune system. Although a role of Hsp70 in inflammation and anti-inflammation has been previously described, the specific ability of exogenous Hsp70 variants to counteract or reverse an immunoparalyzed status has not been described before.

In a first aspect, the invention therefore provides a compound comprising Hsp70 or a therapeutically active part thereof for use in the treatment or prevention of immune suppression in an individual. Also provided is a method for treating or preventing immunoparalysis in an individual in need thereof, comprising administering to the individual a therapeutically effective amount of a compound comprising Hsp70 or a therapeutically active part thereof.

In a further aspect, the invention provides a compound comprising Hsp70 or a therapeutically active part thereof for use in reversing immune suppression in an individual. Also provided is a method for reversing immune suppression in an individual in need thereof, comprising administering to the individual a therapeutically effective amount of a compound comprising Hsp70 or a therapeutically active part thereof.

In a further aspect, the invention provides a compound comprising Hsp70 or a therapeutically active part thereof for use in inducing trained immunity in an individual. Also provided is a method for inducing trained immunity in an individual in need thereof, comprising administering to the individual a therapeutically effective amount of a compound comprising Hsp70 or a therapeutically active part thereof.

Heat shock protein 70 (Hsp70; also *inter alia* referred to as HSPA1A, Hsp72 or HEL-S-103) is a chaperone protein that is expressed in response to stress. Hsp70 binds to its protein substrate and stabilizes it to prevent denaturation or aggregation. In addition, Hsp70 has multiple roles during normal growth, including assisting in folding of newly synthesized proteins, subcellular transport of proteins and vesicles, formation and dissociation of complexes, and degradation of proteins.

Heat shock proteins, including Hsp70, are highly conserved, from microbes to mammals. Hence, the Hsp70 can be Hsp70 from any species. In preferred embodiments, the Hsp70 is a mammalian Hsp70, a nematode Hsp70 or *Drosophila* Hsp70. The mammal is preferably selected from the group consisting of human, mouse, rat, guinea pig, rabbit, hamster, cat, dog, sheep, pig, goat, bovine, and monkey, more preferably the mammal is a human. A nematode as used herein refers to a member of the phylum Nematoda. The nematode is preferably selected from the group consisting of *Setaria, Wuchereria and Brugia* nematodes, including *Setaria cervim, Setaria digitata, Setaria equina, Setaria javensis, Setaria labiatopapillosa, Setaria marshalli, Wuchereria kalimantani, Wuchereria bancrofti, Brugia timori* and *Brugia malayi.* More preferably the nematode is *Setaria digitate, Wuchereria bancrofti* or *Brugia malayi.* In some preferred embodiments, the Hsp70 is human Hsp70 or *Setaria digitata* Hsp70.

The Hsp70 or therapeutically active part thereof has the sequence of wildtype Hsp70 or the therapeutically active part thereof or a sequence that has at least 85% sequence identity therewith, preferably at least 86% sequence identity therewith, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, more preferably at least 99% sequence identity therewith. E.g. a human Hsp70 or therapeutically active part thereof has at least 85% sequence identity with a wildtype human Hsp70 or the part thereof. As another example, a *Setaria digitata* Hsp70 or therapeutically active part thereof has at least 85% sequence identity with a wildtype *Setaria digitata* Hsp70 or the part thereof.

The variation in sequence from wildtype Hsp70 is preferably substitution of one or more amino acids in wildtype Hsp70 by another amino acid. The substitutions are preferably substitutions that do not result in abolition of therapeutic effect, i.e. the treating, counteracting or reversing of immune suppression, in particular immunoparalysis. Substitution may, for instance, result in an enhancement of therapeutic effectivity and/or (storage) stability, prolonged *in vivo* half-life of the Hsp70 or therapeutically active part thereof. Substitution may, however, result in a reduction of the therapeutic efficacy, e.g. to improve other properties such as stability and *in vivo* half-life. A skilled person is well capable of introducing amino acid substitutions in the Hsp70 sequence, while maintaining therapeutic activity. Whether or not therapeutic activity is maintained or maintained to a sufficient degree can be determined by methods for assessing whether Hsp70 or part thereof has the ability to reverse immune suppression in an individual, in particular reversal of immunoparalysis, as described herein below.

In preferred embodiments, the variation in sequence from wildtype Hsp70 is conservative amino acid substitution. Conservative amino acid substitutions are well known in the art and a person skilled in the art is well capable of selecting suitable substitutions for any amino acid. Examples of conservative amino acid substitutions, unlikely to abolish therapeutic activity, include the following: alanine for serine, valine for isoleucine, aspartate for glutamate, threonine for serine, alanine for glycine, alanine for threonine, serine for asparagine, alanine for valine, serine for glycine, tyrosine for phenylalanine, alanine for proline, lysine for arginine, aspartate for asparagine, leucine for isoleucine, leucine for valine, alanine for glutamate, aspartate for glycine, and *vice versa.* Preferably, a conservative substitution is an exchange of one amino acid within a group for another amino acid within the same group, whereby the groups are the following: (1) alanine, valine, leucine, isoleucine, methionine, and phenylalanine: (2) histidine, arginine, lysine, glutamine, and asparagine; (3) aspartate and glutamate; (4) serine, threonine, alanine, tyrosine, phenylalanine, tryptophan, and cysteine; and (5) glycine, proline, and alanine.

Preferably, the Hsp70 or therapeutically active part has at least 85% sequence identity with a wildtype Hsp70 or part thereof, more preferably at least 86%, 87%, 88%, 89%, 90%, 91%, 92%, 93%, 94%, 95%, 06%, 97%, 98% or 99%, maintains the ability to reverse immune suppression in an individual, in particular reversal of immunoparalysis, and/or the ability to prevent or counteractive post-injury infection, secondary infection, infection following trauma as defined herein or complications of such infections in an individual of wildtype Hsp70. E.g. the Hsp70 or therapeutically active part that has at least 85% sequence identity with a wildtype Hsp70 or part thereof, more preferably at least 86%, 87%, 88%, 89%, 90%, 91%, 92%, 93%, 94%, 95%, 06%, 97%, 98% or 99% comprises the ability to increase IL-6 expression by immuneparalyzed monocytes.

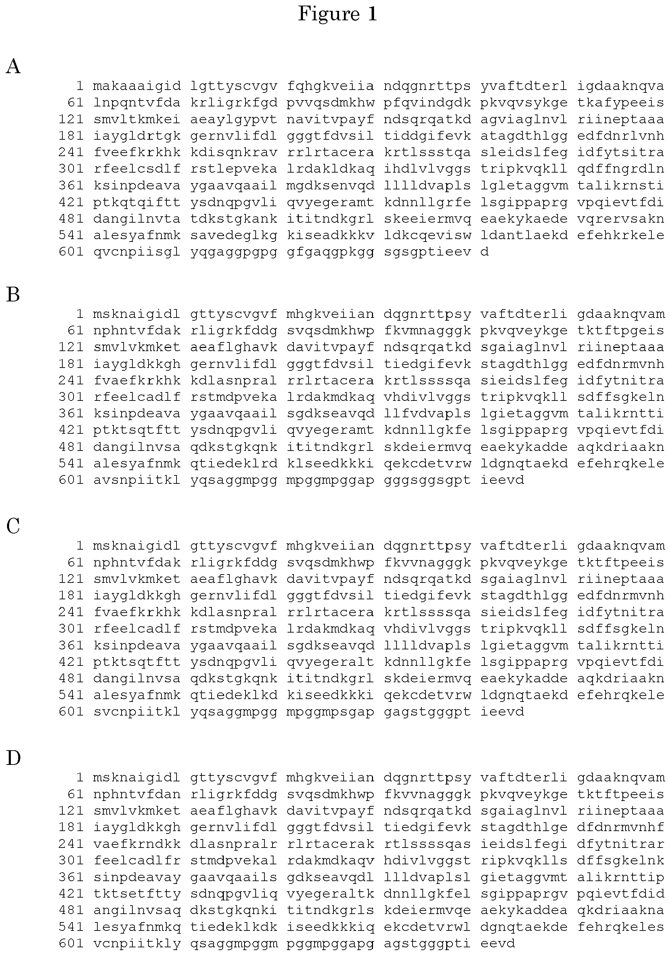

In some preferred embodiments, the Hsp70 or therapeutically active part thereof is human Hsp70 or therapeutically active part thereof as shown in figure 1A or a sequence that has at least 85% sequence identity therewith, more preferably at least 86%, 87%, 88%, 89%, 90%, 91%, 92%, 93%, 94%, 95%, 06%, 97%, 98% or 99% therewith.

In some preferred embodiments, the Hsp70 or therapeutically active part thereof is *Setaria digitata* Hsp70 or therapeutically active part thereof as shown in figure 1B or a sequence that has at least 85% sequence identity therewith, more preferably at least 86%, 87%, 88%, 89%, 90%, 91%, 92%, 93%, 94%, 95%, 06%, 97%, 98% or 99% therewith.

In some preferred embodiments, the Hsp70 or therapeutically active part thereof is *Wuchereria bancrofti* Hsp70 or therapeutically active part thereof as shown in figure 1C or a sequence that has at least 85% sequence identity therewith, more preferably at least 86%, 87%, 88%, 89%, 90%, 91%, 92%, 93%, 94%, 95%, 06%, 97%, 98% or 99% therewith.

In some preferred embodiments, the Hsp70 or therapeutically active part thereof is *Brugia malayi* Hsp70 or therapeutically active part thereof as shown in figure 1D or a sequence that has at least 85% sequence identity therewith, more preferably at least 86%, 87%, 88%, 89%, 90%, 91%, 92%, 93%, 94%, 95%, 06%, 97%, 98% or 99% therewith.

In some preferred embodiments the Hsp70 comprises or is full length Hsp70, preferably mammalian or nematode Hsp70. In some preferred embodiments, the Hsp70 comprises full length Hsp70. In some preferred embodiments, the Hsp70 consists of full length Hsp70.

A "therapeutically active part" of Hsp70 as used herein refers to a part of Hsp70 that has at least one shared property as Hsp70 in kind, although not necessarily in the same amount. The at least one shared property comprises the ability to increase IL-6 expression by immuneparalyzed monocytes, reverse immune suppression in an individual, in particular reversal of immunoparalysis, and/or the ability to prevent or counteractive post-injury infection, secondary infection, infection following trauma or complications of such infections in an individual.

Whether or not a compound comprising a Hsp70 or part of Hsp70 has the ability to reverse immune suppression in an individual, in particular reversal of immunoparalysis, and/or the ability to prevent or counteractive post-injury infection, secondary infection, infection following trauma or complications of such infections in an individual can be determined by a person skilled in the art, e.g. using a method involving an in vitro model as described in the Examples herein. In brief, this model for immunoparalysis involves the induction of endotoxin tolerance in isolated human PBMC's and/or monocytes by incubation with, e.g., *E*. *coli* lipopolysaccharide (LPS) for e.g. 24 hours. Unstimulated cells can be used as control. Following the LPS incubation, and an, e.g. 4 day, resting period, the cells can be incubated with the compound comprising Hsp70 or part thereof or Hsp70 or part thereof, in the presence or absence of LPS. If the compound or Hsp70 or part thereof is able to restore expression of cytokines such as TNFα and/or IL-6 by the cells at least in part to the expression levels of the same cytokines of the control cells, the compound or Hsp70 or part thereof has the ability to reverse immunoparalysis in an individual.

The amino acid sequence of a therapeutically active part preferably comprises or consists of a consecutive stretch of amino acids of the full length Hsp70, or of a sequence that has at least 85% sequence identity therewith, more preferably at least 86%, 87%, 88%, 89%, 90%, 91%, 92%, 93%, 94%, 95%, 06%, 97%, 98% or 99% therewith. A therapeutically active part of a Hsp70 preferably comprises a C-terminal domain of Hsp70. Hsp70 consist of three structural domains: a N-terminal ATPase domain, a substrate binding domain and a C-terminal domain. For human Hsp70, the In preferred embodiments, the therapeutically active part of a Hsp70 preferably comprises or consists of:
- amino acids 508-637 of human Hsp70, preferably of the sequence as depicted in figure 1A, or a sequence that has at least 85% sequence identity therewith, more preferably at least 86%, 87%, 88%, 89%, 90%, 91%, 92%, 93%, 94%, 95%, 06%, 97%, 98% or 99% therewith, or
- amino acids 508-641 of *Setaria digitata* Hsp70, preferably of the sequence as depicted in figure 1B, or a sequence that has at least 85% sequence identity therewith, more preferably at least 86%, 87%, 88%, 89%, 90%, 91%, 92%, 93%, 94%, 95%, 06%, 97%, 98% or 99% therewith, or
- amino acids 508-641 of *Wuchereria bancrofti* Hsp70, preferably of the sequence as depicted in figure 1C, or a sequence that has at least 85% sequence identity therewith, more preferably at least 86%, 87%, 88%, 89%, 90%, 91%, 92%, 93%, 94%, 95%, 06%, 97%, 98% or 99% therewith, or

- amino acids 508-640 of *Brugia malayi* Hsp70, preferably of the sequence as depicted in figure 1D, or a sequence that has at least 85% sequence identity therewith, more preferably at least 86%, 87%, 88%, 89%, 90%, 91%, 92%, 93%, 94%, 95%, 06%, 97%, 98% or 99% therewith, or
- the corresponding amino acids of Hsp70 of another species or a sequence that has at least 85% sequence identity therewith, more preferably at least 86%, 87%, 88%, 89%, 90%, 91%, 92%, 93%, 94%, 95%, 06%, 97%, 98% or 99% therewith.

In other preferred embodiments, the therapeutically active part of a Hsp70 comprises or consist of
- amino acids 508-641 of human Hsp70, preferably of the sequence as depicted in figure 1A, or a sequence that has at least 85% sequence identity therewith, more preferably at least 86%, 87%, 88%, 89%, 90%, 91%, 92%, 93%, 94%, 95%, 06%, 97%, 98% or 99% therewith, or
- amino acids 508-645 of *Setaria digitata* Hsp70, preferably of the sequence as depicted in figure 1B, or a sequence that has at least 85% sequence identity therewith, more preferably at least 86%, 87%, 88%, 89%, 90%, 91%, 92%, 93%, 94%, 95%, 06%, 97%, 98% or 99% therewith, or
- amino acids 508-645 of *Wuchereria bancrofti* Hsp70, preferably of the sequence as depicted in figure 1C, or a sequence that has at least 85% sequence identity therewith, more preferably at least 86%, 87%, 88%, 89%, 90%, 91%, 92%, 93%, 94%, 95%, 06%, 97%, 98% or 99% therewith, or
- amino acids 508-644 of *Brugia malayi* Hsp70, preferably of the sequence as depicted in figure 1D, or a sequence that has at least 85% sequence identity therewith, more preferably at least 86%, 87%, 88%, 89%, 90%, 91%, 92%, 93%, 94%, 95%, 06%, 97%, 98% or 99% therewith, or
- the corresponding amino acids of Hsp70 of another species.

In other preferred embodiments, the therapeutically active part of a Hsp70 comprises or consist of
- amino acids 397-641 of human Hsp70, preferably of the sequence as depicted in figure 1A, or a sequence that has at least 85% sequence identity therewith, more preferably at least 86%, 87%, 88%, 89%, 90%, 91%, 92%, 93%, 94%, 95%, 06%, 97%, 98% or 99% therewith, or
- amino acids 397-645 of *Setaria digitata* Hsp70, preferably of the sequence as depicted in figure 1B, or a sequence that has at least 85% sequence identity therewith, more preferably at least 86%, 87%, 88%, 89%, 90%, 91%, 92%, 93%, 94%, 95%, 06%, 97%, 98% or 99% therewith, or
- amino acids 397-645 of *Wuchereria bancrofti* Hsp70, preferably of the sequence as depicted in figure 1C, or a sequence that has at least 85% sequence identity therewith, more preferably at least 86%, 87%, 88%, 89%, 90%, 91%, 92%, 93%, 94%, 95%, 06%, 97%, 98% or 99% therewith, or
- amino acids 396-644 of *Brugia malayi* Hsp70, preferably of the sequence as depicted in figure 1D, or a sequence that has at least 85% sequence identity therewith, more preferably at least 86%, 87%, 88%, 89%, 90%, 91%, 92%, 93%, 94%, 95%, 06%, 97%, 98% or 99% therewith, or
- the corresponding amino acids of Hsp70 of another species.

In other preferred embodiments, the therapeutically active part of a Hsp70 comprises or consist of
- amino acids 388-641 of human Hsp70, preferably of the sequence as depicted in figure 1A, or a sequence that has at least 85% sequence identity therewith, more preferably at least 86%, 87%, 88%, 89%, 90%, 91%, 92%, 93%, 94%, 95%, 06%, 97%, 98% or 99% therewith, or
- amino acids 388-645 of *Setaria digitata* Hsp70, preferably of the sequence as depicted in figure 1B, or a sequence that has at least 85% sequence identity therewith, more preferably at least 86%, 87%, 88%, 89%, 90%, 91%, 92%, 93%, 94%, 95%, 06%, 97%, 98% or 99% therewith, or
- amino acids 388-645 of *Wuchereria bancrofti* Hsp70, preferably of the sequence as depicted in figure 1C, or a sequence that has at least 85% sequence identity therewith, more preferably at least 86%, 87%, 88%, 89%, 90%, 91%, 92%, 93%, 94%, 95%, 06%, 97%, 98% or 99% therewith, or
- amino acids 387-644 of *Brugia malayi* Hsp70, preferably of the sequence as depicted in figure 1D, or a sequence that has at least 85% sequence identity therewith, more preferably at least 86%, 87%, 88%, 89%, 90%, 91%, 92%, 93%, 94%, 95%, 06%, 97%, 98% or 99% therewith, or
- the corresponding amino acids of Hsp70 of another species.

In other preferred embodiments, the therapeutically active part of Hsp70 comprises at least 10 consecutive amino acids of amino acids 388-641, amino acids 397-641 or amino acids 508-641 of human Hsp70, preferably of the sequence as depicted in figure 1A, or a sequence that has at least 85% sequence identity therewith, more preferably at least 86%, 87%, 88%, 89%, 90%, 91%, 92%, 93%, 94%, 95%, 06%, 97%, 98% or 99% therewith, more preferably at least 15 consecutive amino acids, more preferably at least 20 consecutive amino acids, more preferably at least 25 consecutive amino acids, more preferably at least 30 consecutive amino acids, more preferably at least 35 consecutive amino acids, more preferably at least 40 consecutive amino acids, more preferably at least 45 consecutive amino acids more preferably at least 50 consecutive amino acids, thereof.

In other preferred embodiments, the therapeutically active part of Hsp70 comprises at least 10 consecutive amino acids of the amino acid sequence of a non-human Hsp70 corresponding to amino acids 508-641 of human Hsp70, preferably of the sequence as depicted in figure 1A, or a sequence that has at least 85% sequence identity therewith, more preferably at least 86%, 87%, 88%, 89%, 90%, 91%, 92%, 93%, 94%, 95%, 06%, 97%, 98% or 99% therewith, more preferably at least 15 consecutive amino acids, more preferably at least 20 consecutive amino acids, more preferably at least 25 consecutive amino acids, more preferably at least 30 consecutive amino acids, more preferably at least 35 consecutive amino acids, more preferably at least 40 consecutive amino acids, more preferably at least 45 consecutive amino acids more preferably at least 50 consecutive amino acids, thereof.

In other preferred embodiments, the therapeutically active part of Hsp70 comprises at least 10 consecutive amino acids 388-641, amino acids 397-641 or amino acids 508-641 of *Setaria digitata* Hsp70, preferably of the sequence as depicted in figure 1B, or a sequence that has at least 85% sequence identity therewith, more preferably at least 86%, 87%, 88%, 89%, 90%, 91%, 92%, 93%, 94%, 95%, 06%, 97%, 98% or 99% therewith, more preferably at least 15 consecutive amino acids, more preferably at least 20 consecutive amino acids, more preferably at least 25 consecutive amino acids, more preferably at least 30 consecutive amino acids, more preferably at least 35 consecutive amino acids, more preferably at least 40 consecutive amino acids, more preferably at least 45 consecutive amino acids more preferably at least 50 consecutive amino acids, thereof.

In other preferred embodiments, the therapeutically active part of Hsp70 comprises at least 10 consecutive amino acids 388-641, amino acids 397-641 or amino acids 508-641 of *Wuchereria bancrofti* Hsp70, preferably of the sequence as depicted in figure 1C, or a sequence that has at least 85% sequence identity therewith, more preferably at least 86%, 87%, 88%, 89%, 90%, 91%, 92%, 93%, 94%, 95%, 06%, 97%, 98% or 99% therewith, more preferably at least 15 consecutive amino acids, more preferably at least 20 consecutive amino acids, more preferably at least 25 consecutive amino acids, more preferably at least 30 consecutive amino acids, more preferably at least 35 consecutive amino acids, more preferably at least 40 consecutive amino acids, more preferably at least 45 consecutive amino acids more preferably at least 50 consecutive amino acids, thereof.

In other preferred embodiments, the therapeutically active part of Hsp70 comprises at least 10 consecutive amino acids 387-641, amino acids 396-641 or amino acids 507-641 of *Brugia malayi* Hsp70, preferably of the sequence as depicted in figure 1D, or a sequence that has at least 85% sequence identity therewith, more preferably at least 86%, 87%, 88%, 89%, 90%, 91%, 92%, 93%, 94%, 95%, 06%, 97%, 98% or 99% therewith, more preferably at least 15 consecutive amino acids, more preferably at least 20 consecutive amino acids, more preferably at least 25 consecutive amino acids, more preferably at least 30 consecutive amino acids, more preferably at least 35 consecutive amino acids, more preferably at least 40 consecutive amino acids, more preferably at least 45 consecutive amino acids more preferably at least 50 consecutive amino acids, thereof.

The therapeutically active part of Hsp70 may comprise additional amino acids. In preferred embodiments, these additional amino acids are part of the full length Hsp70 of the same species, or of a sequence that has at least 85% sequence identity therewith, more preferably at least 86%, 87%, 88%, 89%, 90%, 91%, 92%, 93%, 94%, 95%, 06%, 97%, 98% or 99% therewith. In further preferred embodiments, these additional amino acids are such that the amino acids sequence of the therapeutically active part is a consecutive stretch of amino acids of the full length Hsp70 of the same species, or of a sequence that has at least 85% sequence identity therewith, more preferably at least 86%, 87%, 88%, 89%, 90%, 91%, 92%, 93%, 94%, 95%, 06%, 97%, 98% or 99% therewith.

In other preferred embodiments, a Hsp70 or therapeutically active part of Hsp70 may comprise additional amino acids, whereby the additional amino acids are not derived from a Hsp70 protein. It is preferred that at most 1-50 of such non-Hsp70 amino acids are comprised in the Hsp70 or therapeutically active part thereof.

The Hsp70 or therapeutically active part thereof may comprise modifications, in particular N-terminal, C-terminal modifications and/or internal modifications, such as C-terminal amidation, N-terminal acylation, fatty acid modifications, esterification and combinations thereof. Internal modifications include the presence of non-naturally occurring amino acids and D amino acids, wherein the non-naturally occurring amino acids and D amino acids are variants of or correspond to the original amino acid.

The Hsp70 or therapeutically active part thereof may be comprised in a compound. A compound according to the invention preferably comprises a further moiety such as a label, such as an imaging label or detection label, a linker, such as a PEG linker, or a targeting moiety coupled to the Hsp70 or therapeutically active part thereof or said compound comprises the Hsp70 or therapeutically active part thereof coupled to or encapsulated into a carrier or targeting moiety. In some embodiments, a carrier or targeting moiety is selected from the group consisting of nanoparticles, microparticles, nanocapsules, nanocomplexes, polyplexes, carbon nanotubes, quantum dots, microcapsules, liposomes, microspheres, hydrogels, polymers, micelles, dendrimers, lipid complexes, serum albumin, antibodies, antibody fragments, cyclodextrins and dextran. In some embodiments, the compound according to the invention is the Hsp70 or therapeutically active part thereof.

The Hsp70 or therapeutically active part thereof may be in the form of a pharmaceutically acceptable salt. Such salts include, but are not limited to, acid addition salts and base addition salts. As used herein, "pharmaceutically acceptable salt" of a protein or peptide refers to a salt that retains the desired activity of the protein or peptide, and is suitable for administration to humans or animals. Examples of pharmaceutically acceptable acids and bases include organic and inorganic acids such as formic acid, acetic acid, propionic acid, lactic acid, glycolic acid, oxalic acid, pyruvic acid, succinic acid, maleic acid, malonic acid, trifluoroacetic acid, cinnamic acid, sulfuric acid, hydrochloric acid, hydrobromic acid, nitric acid, perchloric acid, phosphoric acid, and thiocyanic acid, which form ammonium salts with free amino groups of protein or peptide, and bases which form carboxylate salts with free carboxylic groups of protein or peptide, such as ethylamine, methylamine, dimethylamine, triethylamine, isopropylamine, diisopropylamine, and other mono-, di-and trialkylamines, and arylamines.

The Hsp70 or therapeutically active part thereof or pharmaceutically acceptable salt thereof, or compound comprising the same, used in accordance with the invention may further be comprised in a pharmaceutical composition. The pharmaceutical composition further preferably comprises at least one pharmaceutically acceptable carrier, diluent and/or excipient. By "pharmaceutically acceptable" it is meant that the carrier, diluent or excipient must be compatible with the other ingredients of the composition and not deleterious, e.g. toxic, to the recipient thereof. In general, any pharmaceutically suitable additive which does not interfere with the function of the compound or Hsp70 or therapeutically active part thereof can be used.

Compositions for parenteral administration, such as intravenous administration, may for example be solutions of the compound or Hsp70 or part thereof in sterile isotonic aqueous buffer. Where necessary, the compositions may include for instance solubilizing agents, stabilizing agents, antimicrobial agents and/or a local anesthetic to ease the pain at the site of the injection.

The compound or Hsp70 or therapeutically active part thereof may be administered enterally or parenterally. Parenteral administration can include, for example, intraarticular, intramuscular, intravenous, intraventricular, intraarterial, intrathecal, intranasal, intradermal, subcutaneous, or intraperitoneal administration. Further, the compound or Hsp70 or therapeutically active part thereof, may be administered to an individual in a hospital or other health care facility via infusion or via injection by a healthcare professional. The exact dose and regimen of the compound or Hsp70 or therapeutically active part thereof and compositions thereof will be dependent on the biological activity of the protein or part thereof per se, the age, weight and sex of the individual, the needs of the individual to whom the medicament is administered, the degree of affliction or need and the judgment of the medical practitioner.

In embodiments, a compound comprising Hsp70 or a therapeutically active part thereof as defined herein is used in the treatment or prevention of or reversing an immune suppression in an individual. In embodiments, a compound comprising Hsp70 or a therapeutically active part thereof as defined herein is used in inducing trained immunity in an individual. As used herein "immune suppression", also known as "immunosuppression" refers to a reduced efficacy of the immune system, in particular to a reduced inflammatory response and ability to fight invading pathogens. Immune suppression can be caused by a variety of disorders and conditions, including, but not limited to, cancer, complement deficiencies, infections, sepsis, and the use of drugs, including immunosuppressant drugs. As used herein "trained immunity" refers to an ability of the innate immune cells to functionally reprogram after an endogenous of exogenous or endogenous insult and subsequently respond, non-specifically, to restimulation. It further refers to a functional state of the innate immune response which is characterized by epigenetic reprogramming of innate immune cells. Training of innate immune cells enhances immune responses against microbial pathogens after restimulation.

Persistence of a marked compensatory anti-inflammatory innate immune response is termed immunoparalysis. In this state, the severely underactive immune system is unable to detect pathogens, mount an inflammatory response, destroy microbial invaders, or repair damaged tissue places the patient at risk for death from secondary infection and persistent organ failure. The present inventors have surprisingly found that Hsp70 or a part thereof is able to reverse not only immune suppression but also immunoparalysis. Hence, in preferred embodiments, the compound or Hsp70 or therapeutically active part thereof is for use in the treatment or prevention of immunoparalysis. Immunoparalysis is a condition well known in the art and is also referred to as immunological paralysis, immune paralysis, immune paralytic syndrome and endotoxin tolerance. Immunoparalysis is essentially the ultimate form of immune suppression. Immunoparalysis is characterized by a decrease in total lymphocytes, a decrease in the expression of cytokines like tumor necrosis factor alpha (TNF-α) and interleukin-6 (IL-6), interferon gamma (IFN-y), and high mobility group box 1 protein (HMGB1) , increase in IL-10 and reduced expression of human leukocyte antigen (HLA) DR (HLA-DR) by monocytes and dendritic cells.

In preferred embodiments, immune suppression in an individual is reversed.

In preferred embodiments, immunoparalysis in an individual is reversed.

The presence or absence of immunoparalysis in an individual can be established by any method known in the art, e.g. based on current standards for determining immunoparalysis. In particular, clinical presentation of the state of immunoparalysis and/or the presence of biomarkers for immunoparalysis can be determined. Clinical presentation of immunoparalysis for instance comprises the presence of a secondary infection. Cajander et al. (2024), which is incorporated by reference herein describes markers for the presence of immunoparalysis, referred to as a severe dysregulation immune phenotype therein, in patients suffering from sepsis. These markers are equally useful in determining immunoparalysis in individuals or patients suffering from other disease, disorders or conditions.

Examples of markers for immunoparalysis are the measurement of low cell-surface expression of HLA-DR on monocytes isolated from the individual, through analysis of the capacity of whole blood isolated from the individual to produce TNFα upon *ex vivo* stimulation with endotoxin, such as lipopolysaccharide (LPS) or other microbial antigens, PHA or Anti-CD2/CD28, high expression of L-10, high soluble programmed cell death1 ligand 1(sPD-L1), high expression of transforming growth factor-β (TGF- β), low expression of INF-gamma, low expression of HMGB1, the measurement of low total lymphocyte count in blood, determination of viral reactivation of latent viral infections, high-throughput omics data (combitypes) to define endotypes of clinical utility (e.g. the immunological, inflammatory, metabolic, and remodeling pathways that explain the mechanisms underlying a clinical presentation (phenotype) of the disease), and combinations thereof.

Detection of HLA-DR may be performed by detecting the cell surface protein in a quantitative manner for instance by means of affinity based reactions using flow cytometry, lateral flow assays, photonic biochips etc. but also by genetic profiling such as PCR or sequencing technologies. Expression and presence of INF-gamma, IL10 and HMGB1 can be detected by common affinity based serological tests, cytokine panels, DAMP panels, lateral flow tests, photonic biochips etc. but also by genetic profiling such as PCR or sequencing technologies. Such expression is for instance compared with a reference. A reference is for instance, or is for instance based on, the expression of the same cytokine by monocytes or whole blood isolated from healthy individual(s) or expression of HLA-DR on the cell surface of monocytes isolated from healthy individual(s).

In some preferred embodiments of the methods and uses of the invention, the individual is diagnosed as suffering from immunoparalysis.

In preferred embodiments, determining whether an individual suffers from immunoparalysis comprises assessing clinical presentation of immunoparalysis and/or determining at least one of the following markers:
- cell-surface expression of HLA-DR on monocytes isolated from the individual;
- the capacity of whole blood isolated from the individual to produce TNFα upon *ex vivo* stimulation with endotoxin, such as lipopolysaccharide (LPS) or other microbial antigens, PHA or Anti-CD2/CD28;
- expression of L-10;
- soluble programmed cell death 1 ligand 1 (sPD-L1) concentration;
- transforming growth factor-β (TGF- β) expression;
- INF-gamma expression;
- HMGB1 expression;
- total lymphocyte blood count;
- determination of viral reactivation and/or endotype and/or combitype of latent viral infections,
- high-throughput omics data (combitypes) to define endotypes of clinical utility (e.g. the immunological, inflammatory, metabolic, and remodeling pathways that explain the mechanisms underlying a clinical presentation (phenotype) of the disease), and
- combinations thereof.

In preferred embodiments, determining whether an individual suffers from immunoparalysis comprises determining at least two of these markers, more preferably at least three of these markers, more preferably at least four, five or six of these markers.

In preferred embodiments, determining whether an individual suffers from immunoparalysis comprises at least determining cell surface expression of HLA-DR on monocytes isolated from the individual.

Preferably, the determined marker value is compared with a reference. As used herein, the term "reference" refers to a reference value for the same marker, e.g. the value of the marker determined in a healthy individual or individuals, i.e. individual not suffering from immunoparalysis, or the individual itself prior to the diagnosis of immunoparalysis. The reference can be a reference obtained from a single individual, and/or the reference value can be the value of the particular marker in a sample of a single individual. It is, however, preferred, that the reference value is the average of the value of a particular marker in a plurality of individuals.

An individual that is treated with a compound or Hsp70 or a therapeutically active part thereof in accordance with the invention is suffering from immune suppression, in particular from immunoparalysis.

Immune suppression, in particular immunoparalysis, is associated with or resulting from a number of diseases, disorders and conditions. The term "resulting from" or "associated with" means that the immune suppression or immunoparalysis is caused, at least in part, by the recited disease, disorder or condition or by the response of the immune system to the recited disease, disorder or condition. In particular, (severe) immune suppression and immunoparalysis can occur in individuals suffering from trauma. As used herein "trauma" refers to a severe physiological stress event or condition. "Physiological stress" as used herein refers to any external or internal condition that challenges the homeostasis of an individual. Examples of severe physiological stress events and conditions include for instance shock, hypoxemia, acidosis, sepsis, injury, burns, major surgery, organ failure and multiple organ system failure. Such severe physiological stress event or condition is in particular potentially life-threatening. I.e. "trauma" as used herein preferably refers to a potentially life-threatening physical stress event or condition. More some embodiments, trauma concerns critical illness, which refers to a medical condition in which an individual, because of e.g. major surgery or severe illness, requires immediate intensive medical support of vital organ functions in order to survive.

Hence, in preferred embodiments, the individual treated with a compound or Hsp70 or a therapeutically active part thereof in accordance with the invention is suffering from trauma. In particular, the immune suppression, in particular immunoparalysis, is associated with or resulting from the trauma.

Examples of trauma in accordance with the present invention are bacterial infection, viral infection, fungal infections, critical illness, chronic psychological stress, sepsis, septic shock, multiple organ dysfunction syndrome (MODS), (severe) physical injury, stroke, such as acute ischemic stroke, spinal cord injury, liver ischemia-reperfusion injury, myocardial infarction, cystic fibrosis, acute coronary syndrome (ACS), pancreatitis, and/or cancer, and/or has undergone surgery, organ or tissue transplantation, anticancer therapy, chemotherapy, immunosuppressant therapy, long term hospitalisation and/or Intensive Care Unit (ICU) admission.

These diseases, conditions and disorders are all characterized in that a subset of the patients is afflicted with immune suppression, in particular immunoparalysis. The immune suppression, in particular immunoparalysis, treated in accordance with the invention is in particular associated with, caused by or a complication of the trauma. With the present invention it has become possible to selectively treat a subpopulation of patients suffering from trauma as defined herein, in particular suffering from or having undergone one or more of the listed diseases, conditions and disorders. The treatment preferably comprises counteracting or reversing the immune suppression, in particular immunoparalysis. With such treatment, the survival of such patients is for instance improved, preventing or reducing duration of life support and/or preventing long term adverse health effects, as compared to patients who did not receive the treatment in accordance with the present invention.

In some preferred embodiments, the trauma is selected from the group consisting of critical illness, chronic psychological stress, (severe) physical injury, stroke, such as acute ischemic stroke, spinal cord injury, liver ischemia-reperfusion injury, myocardial infarction, cystic fibrosis, acute coronary syndrome (ACS), pancreatitis, and/or cancer, and/or results from surgery, organ or tissue transplantation, anticancer therapy, immunosuppressing therapy, chemotherapy, long term hospitalization and/or Intensive Care Unit (ICU) admission.

In some preferred embodiments, the trauma is selected from the group consisting of critical illness, chronic psychological stress, (severe) physical injury, stroke, such as acute ischemic stroke, spinal cord injury, liver ischemia-reperfusion injury, myocardial infarction, acute coronary syndrome (ACS), and/or cancer, and/or results from surgery, organ or tissue transplantation, anticancer therapy, chemotherapy, long term hospitalization and/or Intensive Care Unit (ICU) admission.

In some preferred embodiments, the individual is suffering from bacterial infection, viral infection, critical illness, chronic psychological stress, sepsis, septic shock, multiple organ dysfunction syndrome (MODS), (severe) physical injury, stroke, such as acute ischemic stroke, spinal cord injury, liver ischemia-reperfusion injury, myocardial infarction, cystic fibrosis, acute coronary syndrome (ACS), pancreatitis, and/or cancer, and/or has undergone surgery, organ or tissue transplantation, anticancer therapy, chemotherapy, immunosuppressant therapy, long term hospitalisation and/or Intensive Care Unit (ICU) admission, and suffering from immune suppression, more in particular immunoparalysis.

The surgery is in particular a major surgical procedure, cardiopulmonary bypasses or coronary artery bypass grafting. As used herein, major surgery refers to any surgical procedure that involves anaesthesia and/or respiratory assistance. Examples of major surgical procedures include amputations, organ transplant, removal of a tumours, such as brain tumor, removal of a damaged organ or tissue, such as liver, lung and kidney, abdominal surgery, surgery of the digestive tract, surgery of the endocrine system, surgery of the nervous system, surgery of the circulatory system, open-heart surgery, and surgery to repair damage from injury, including injury cause by accidents or burns.

In some preferred embodiments, the anticancer therapy is chemotherapy, radiation therapy or immune therapy.

In some preferred embodiments, the immunoparalysis is not associated with or caused by immunosuppressant therapy. In some preferred embodiments, the individual is not receiving or has not received immunosuppressant therapy for the disease, disorder or condition.

In some preferred embodiments, the individual is not suffering from sepsis.

In some preferred embodiments, the individual is not suffering from sepsis or a sepsis-related disorder, such as septic shock, MODS and/or systemic inflammatory response syndrome (SIRS).

In some preferred embodiments, the individual is suffering from immunoparalysis and critical illness, (severe) physical injury, stroke, such as acute ischemic stroke, liver ischemia-reperfusion injury, myocardial infarction, acute coronary syndrome (ACS), pancreatitis, and/or cancer, and/or has undergone surgery, organ or tissue transplantation, anticancer therapy, chemotherapy, long term hospitalisation and/or Intensive Care Unit (ICU) admission.

In preferred embodiments, the individual is suffering from cancer and is receiving or has received anticancer therapy, including chemotherapy, radiation therapy and immune therapy.

In some preferred embodiments, the individual is suffering from sepsis.

In some preferred embodiments, the individual is suffering from sepsis and/or a sepsis-related disorder, such as septic shock, MODS and/or systemic inflammatory response syndrome (SIRS).

Examples of complications of trauma include, but are not limited to, secondary infection, sepsis, organ failure, including multiple organ failure, amputations, loss of body functions, brain damage, atelectasis, disseminated intravascular coagulation, coagulation disorder, and, ultimately, death.

In preferred embodiments, the survival of the individual suffering from trauma is improved by the treatment with a compound or Hsp70 or therapeutically active part thereof in accordance with the invention. "Improved survival" refers to the improvement in the survival rate of the individual treated with a compound or Hsp70 or a therapeutically active part thereof compared to an individual that is not treated in accordance with the invention.

In preferred embodiments, quality of life of the individual suffering from trauma is improved by the treatment with a compound or Hsp70 or therapeutically active part thereof in accordance with the invention. "Quality of life" refers to the degree of physical, emotional, and social wellbeing of the individual that is treated in accordance with the present invention.

In preferred embodiments, the need for life support is avoided or the duration thereof reduced and/or long term adverse health effects are prevented or reduced.

In some embodiments, the invention relates to prevention of immune suppression, in particular immunoparalysis, and the individual is suffering from trauma as defined herein and at risk of suffering from immunoparalysis.

As indicated herein above, not all patients suffering from trauma as defined herein will develop or suffer from immunoparalysis. Hence, the invention further provides a compound comprising Hsp70 or a therapeutically active part thereof as defined herein for use in treating trauma and/or complications thereof in an individual, preferably characterized in that the individual is suffering from immunoparalysis.

In some embodiments, the invention comprises determining whether an individual is suffering from immune suppression or immunoparalysis, in particular from immunoparalysis, to identify the patient population that will or may benefit from treatment with a compound or Hsp70 or a therapeutically active part thereof in accordance with the invention. Said determining preferably comprises assessing clinical presentation of immunoparalysis and/or determining at least one of the following markers:
- cell-surface expression of HLA-DR on monocytes isolated from the individual;
- the capacity of whole blood isolated from the individual to produce TNFα upon *ex vivo* stimulation with endotoxin, such as lipopolysaccharide (LPS) or other microbial antigens, PHA or Anti-CD2/CD28;
- expression of L-10;
- soluble programmed cell death 1 ligand 1 (sPD-L1) concentration;
- transforming growth factor-β (TGF- β) expression;
- INF-gamma expression;
- HMGB1 expression;
- total lymphocyte blood count;
- determination of viral reactivation and/or endotype and/or combitype of latent viral infections,
- high-throughput omics data (combitypes) to define endotypes of clinical utility (e.g. the immunological, inflammatory, metabolic, and remodeling pathways that explain the mechanisms underlying a clinical presentation (phenotype) of the disease), and
- combinations thereof.

In preferred embodiments, determining whether an individual suffers from immunoparalysis comprises determining at least two of these markers, more preferably at least three of these markers, more preferably at least four, five or six of these markers.

In preferred embodiments, determining whether an individual suffers from immunoparalysis comprises at least determining cell surface expression of HLA-DR on monocytes isolated from the individual.

Preferably, the determined marker value is compared with a reference, as defined herein above.

In some preferred embodiments, the individual is diagnosed as suffering from immunoparalysis.

Individuals suffering from a severe physiological stress event or condition, in particular from trauma as defined herein, are often afflicted by additional events, such as infection or inflammation. Because immunoparalysis renders the immune system unresponsive to such additional insult, also referred to as secondary insult, individuals suffering from immunoparalysis are in particular at risk of adverse effect or complications of such secondary insults, including at risk of prolonged hospitalization, ICU admission and increased mortality. Examples of secondary insults are secondary inflammation and secondary infection. A used herein, a "secondary insult" refers to an insult, such as an infection, that develops in an individual following the initial trauma as defined herein. In particular, the trauma is different from said insult. The term "secondary infection" refers to an infection that develops in an individual following trauma as defined herein. In particular, the trauma is different from said infection and, if the trauma is or comprises an infection, the secondary infection is an infection unrelated to any original infection, e.g. by a different bacteria, fungus or virus. Such secondary infection can be any bacterial, fungal or viral infection. The trauma may be, but is not necessarily, an infection.

Hps70 or a therapeutically active part thereof, by reversing immunoparalysis as demonstrated by the present invention, is therefore able to prevent, treat and/or counteract such secondary insult and the consequences thereof. Hence, also provided is a compound comprising Hsp70 or a therapeutically active part thereof for use in preventing or counteracting post-trauma infection, including post-injury infection, secondary infection, post-surgery infection or a complication of such post-trauma infection in an individual. Also provided is a method for preventing, treating or counteracting post-trauma infection, such as post-injury infection, secondary infection or post-surgery infection, or a complication of such post-trauma infection in an individual in need thereof, preferably by counteracting immunoparalysis, comprising administering to the individual a therapeutically effective amount of a compound comprising Hsp70 or a therapeutically active part thereof. Examples of complication of post-trauma infection, in particular of post-injury infection, secondary infection or post-surgery infection are sepsis, organ failure, including multiple organ failure, amputations, loss of body functions, brain damage, atelectasis, disseminated intravascular coagulation, coagulation disorder, and, ultimately, death.

In some embodiments, the individual in suffering from cancer. In particular the immune suppression, in particular immunoparalysis, is associated with or results from cancer, e.g. caused by the cancer or a treatment thereof and/or the response of the immune system thereto. In addition, it is now also known that the suppressed or paralyzed immune system can act as a key barrier for cancer immunotherapy. The suppressed or paralyzed immune system may prevent the body from mounting an appropriate immune reaction in response to the immunotherapy and thereby create favorable conditions for cancer development and progression. Combination of treatment with a compound comprising Hsp70 or therapeutically active part thereof in accordance with the invention with other immune therapeutic agents may be beneficial. Hence, in preferred embodiments, in particular wherein the individual is suffering from cancer and in particular wherein the individual is receiving or has received cancer immunotherapy, treatment with a compound comprising Hsp70 or a therapeutically active part thereof in accordance with the invention is combined with other immune therapeutic agent. Such other immune therapeutic agent include, but are not limited to, therapeutic immune system modulators, cancer vaccines (incl. neoantigens), (monoclonal) anti-cancer antibodies, bispecific or multispecific anti-cancer antibodies, conjugated antibodies, check-point inhibitors, cytokines (e.g. interleukin, interferon), polysaccharides, cell therapy such as CAR-T therapy, TIL (tumor infiltrating lymphocyte) therapy, T-cell receptor gene therapy, adoptive T-cell therapy, T-cell transfer therapy, dendritic cell therapy, oncolytic viruses.

In addition, because of compensatory immune evasion pathways, combination treatment with agents targeting other immune targets may be beneficial. Examples of such immune targets include, but are not limited to, immune checkpoints such as programmed cell death protein 1 (PD-1) / Programmed cell death protein 1 ligand 1 (PD-L1), indoleamine 2,3-Dioxygenase (IDO), Tryptophan 2,3-Dioxygenase (TDO), cytotoxic T lymphocyte antigen-4 (CTLA-4) Lymphocyte Activation Gene-3 (LAG-3), T-Cell Immunoglobulin and ITIM Domain (TIGIT), T-Cell Immunoglobulin- and Mucin-Domain-Containing Molecule-3 (TIM-3), -Domain Immunoglobulin Suppressor of T-Cell Activation (VISTA), CD27/CD70, CD39/CD73, herpesvirus entry mediator / B- and T-lymphocyte attenuator / CD160 (HVEM/BTLA/CD160) and B7 Homolog 3 Protein (B7-H3) and regulatory T cells (Treg). Therapies targeting such immune checkpoints and other immune targets are well known. Checkpoint inhibitors included for instance antibodies. E.g. anti-PD1/PDL1 or anti-CTLA-4 treatment for instance includes treatment with antibodies directed against PD1/PDL1 or CTLA-4. IDO/TDO inhibitors and Treg depletion therapy is are also well known. IDO and TDO inhibitors include small molecules such as navoximod, epacadostat, linrodostat, EOS200271, KHK2455, NTRC 3531-0. Treg depletion is for instance achieved by antibodies directed against OX40 and CD27.

Also provided is a compound comprising Hsp70 or a therapeutically active part as defined herein thereof for use in improving the efficacy of anticancer therapy in an individual. Also provided is a method for improving the efficacy of anticancer therapy in an individual comprising administering to the individual a therapeutically effective amount of the compound or Hsp70 or a therapeutically active part thereof as defined herein.

The Hsp70 protein and parts thereof, including peptides, can be prepared by various methods. For instance, a peptide can be synthesized by commonly used solid-phase synthesis methods, e.g. methods that involve t-BOC or FMOC protection of alpha-amino groups which are well known in the art. Here, amino acids are sequentially added to a growing chain of amino acids. Such methods are for instance described in Merrifield (1963), J. Am. Chem. Soc. 85: 2149-2156 ; and Atherton et al., "Solid Phase Peptide Synthesis," IRL Press, London, (1989). Solid-phase synthesis methods are particularly suitable for synthesis of peptides or relatively short length, such as peptides with a length of up to about 70 amino acids in large-scale production.

Alternatively, a Hsp70 protein or peptide can be prepared using recombinant techniques well known in the art in which a nucleotide sequence encoding the peptide is expressed in host cells. Expression is for instance achieved using a vector, such as a plasmid, bacteriophage or animal virus, capable of introducing a heterologous nucleic acid sequence into a host cell. A vector allows the expression or production of a Hsp70 protein, polypeptide or peptide encoded by the nucleic acid sequence in a host cell. A vector is for instance derived from an animal virus, examples of which include, but not limited to, vaccinia virus (including attenuated derivatives such as the Modified Vaccinia virus Ankara, MVA), Newcastle Disease virus (NDV), adenovirus or retrovirus. A vector may comprise an expression cassette comprising a promoter that is suitable for initiation of transcription of a peptide according to the invention in the selected host cells. Examples of suitable promoters for expression of peptides according to the invention in eukaryotic host cells include, but are not limited to, beta-actin promoter, immunoglobin promoter, 5S RNA promoter, or virus derived promoters such as cytomegalovirus (CMV), Rous sarcoma virus (RSV) and Simian virus 40 (SV40) promoters for mammalian hosts.

Transformation of a host cell can result in transient expression of a recombinant protein or peptide by said cell, meaning that the recombinant protein or peptide is only expressed for a defined period of time. Alternatively, transformation of a recipient cell can result in stable expression, meaning that the nucleic acid is introduced into the genome of the cell and thus passed on to next generations of cells. Additionally, inducible expression of a recombinant protein or peptide can be achieved. An inducible expression system requires the presence or absence of a molecule that allows for expression of a nucleic acid sequence encoding a peptide of the invention. Examples of inducible expression systems include, but are not limited to, Tet-On and Tet-Off expression systems, hormone inducible gene expression system such as for instance an ecdysone inducible gene expression system, an arabinose-inducible gene expression system, and a Drosophila inducible expression system using a pMT/BiP vector (Invitrogen) which comprises an inducible metallothioneine promoter. A host cell is for instance a Gram-positive prokaryote, a Gram-negative prokaryote or an eukaryote, such as a plant cell, a yeast cell, a mammalian cell or an insect cell. Examples of suitable host cells include plant cells such as corn cells, rice cells, duckweed cells, tobacco cells (such as BY-2 or NT-1 cells), and potato cells. Examples of yeast cells are *Saccharomyces* and *Pichia.* Examples of insect cells are *Spodoptera frugiperda* cells, such as Tn5, SF-9 and SF-21 cells, and Drosophila cells, such as Drosophila Schneider 2 (S2) cells. Examples of mammalian cells that are suitable for expressing a peptide according to the invention include, but are not limited to, African Green Monkey kidney (Vero) cells, baby hamster kidney (such as BHK-21) cells, Human retina cells (for example PerC6 cells), human embryonic kidney cells (such as HEK293 cells), Madin Darby Canine kidney (MDCK) cells, Chicken embryo fibroblasts (CEF), Chicken embryo kidney cells (CEK cells), blastoderm-derived embryonic stem cells (e.g. EB14), mouse embryonic fibroblasts (such as 3T3 cells), Chinese hamster ovary (CHO) cells , and derivatives of these cell types.

Features may be described herein as part of the same or separate aspects or embodiments of the present invention for the purpose of clarity and a concise description. It will be appreciated by the skilled person that the scope of the invention may include embodiments having combinations of all or some of the features described herein as part of the same or separate embodiments.

The invention will be explained in more detail in the following, non-limiting examples.

### Brief description of the drawings

Figure 1: **Amino acid sequence** of human (A), *Setaria digitata* (B), *Wuchereria bancrofti* (C) and *Brugia malayi* (D) Hsp70, GenBank accession numbers NP_005336.3, AAD13154.1, EJW86287.1 and AAC17926.1, respectively.

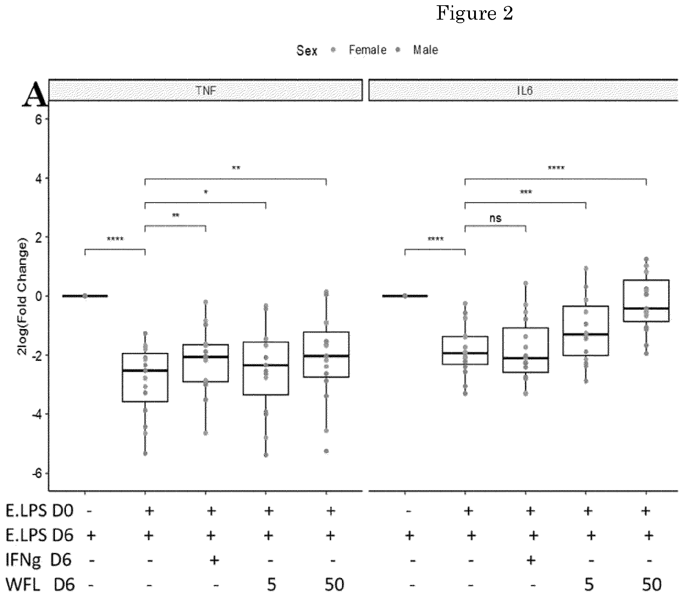

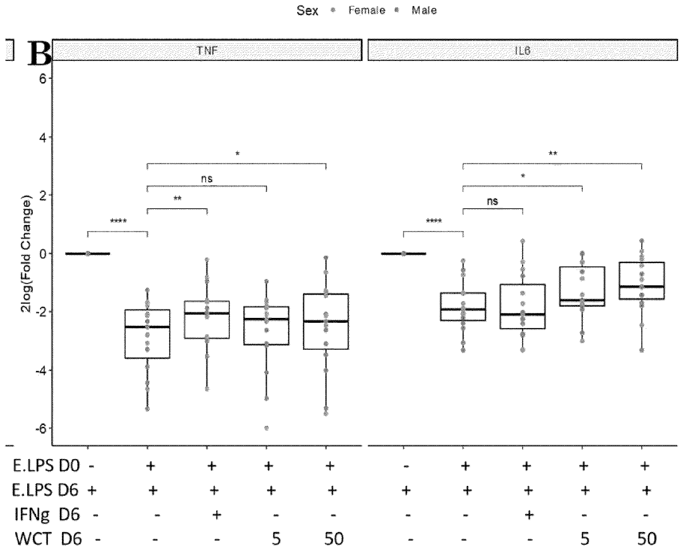

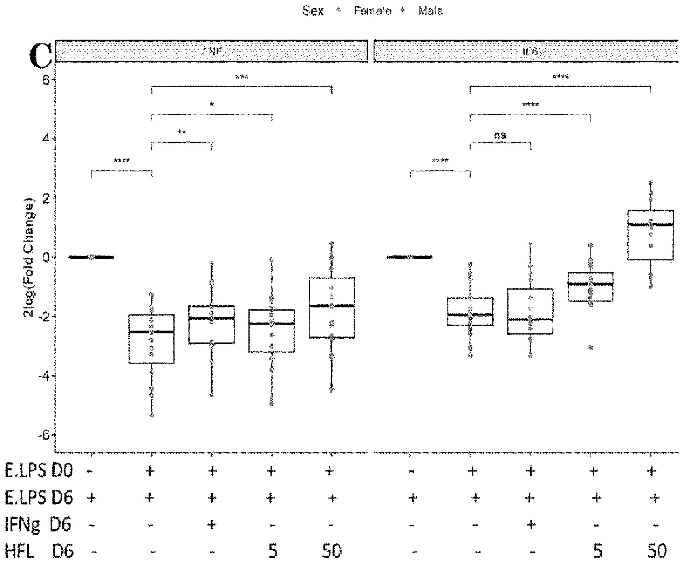

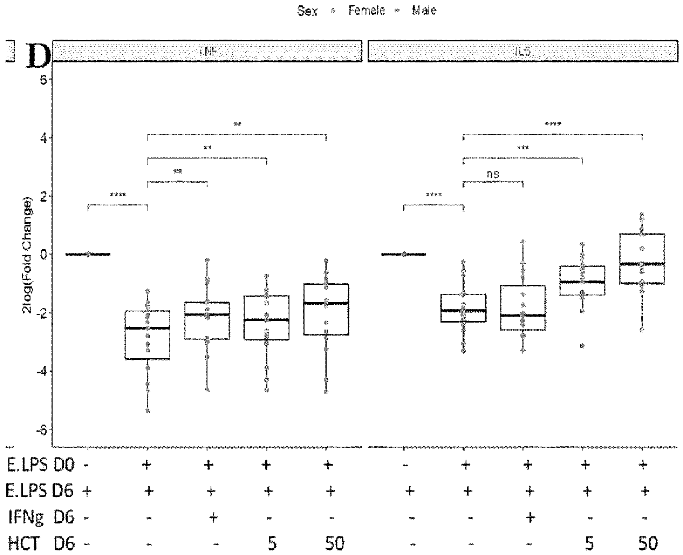

**Figure 2****: Tumor Necrosis Factor alpha (TNF) and interleukin- 6 (IL-6) production by naive and LPS-tolerant percoll monocytes incubated with different reversal agents.** On day 0 (D0), cells were incubated for 24-hours with RPMI (E.LPS D0 -) or *E.coli* LPS 1 ng/mL on day 0 (E.LPS D0 +) for 24 hours. On day 6, naive or LPS-tolerant monocytes were restimulated for 24 hours with *E.coli* LPS 10 ng/mL (E.LPS D6) in the presence and absence of the following reversal stimuli: Interferon-gamma 10 ng/mL (IFNg D6, panel **A-D**), recombinant filarial HSP70 full-length (WFL D6, panel **A**), recombinant filarial HSP70 C-terminal domain (WCT D6, panel **B**), recombinant human HSP70 full-length (HFL D6, panel **C**), recombinant human HSP70 C-terminal domain (HCT D6, panel **D**). Data are represented as box and whiskers showing the log2 fold change in cytokine response compared with naive monocytes stimulated with LPS on day 6. Concentrations depicted in the figures are in nM. Statistical testing was performed using Wilcoxon signed rank tests. * P < 0.05, ** P < 0.01, *** P <0.001, **** P <0.0001.

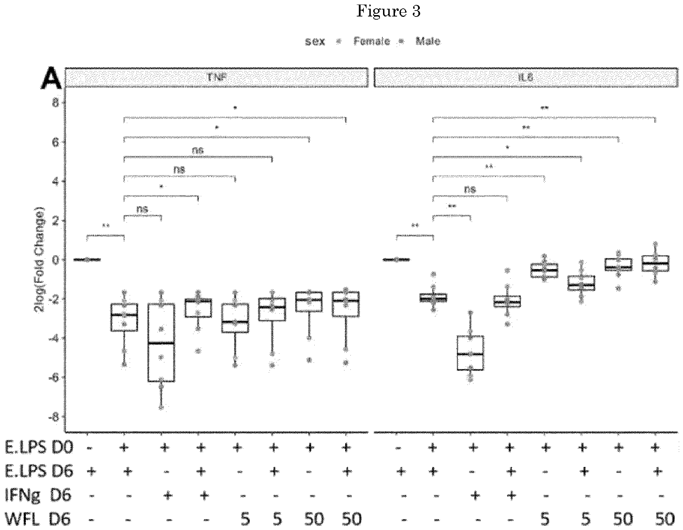

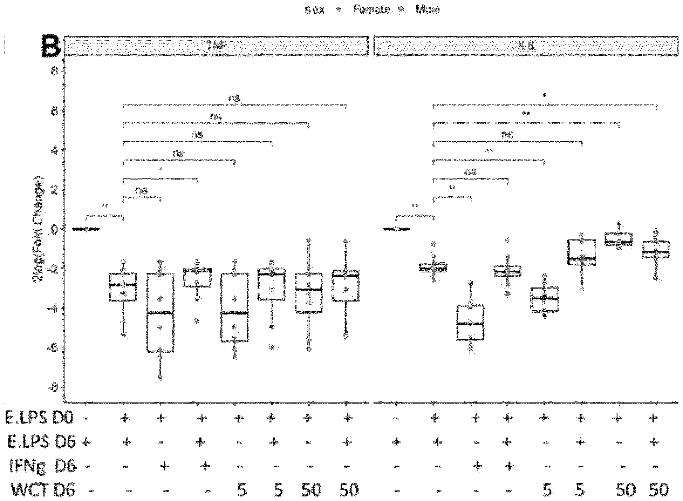

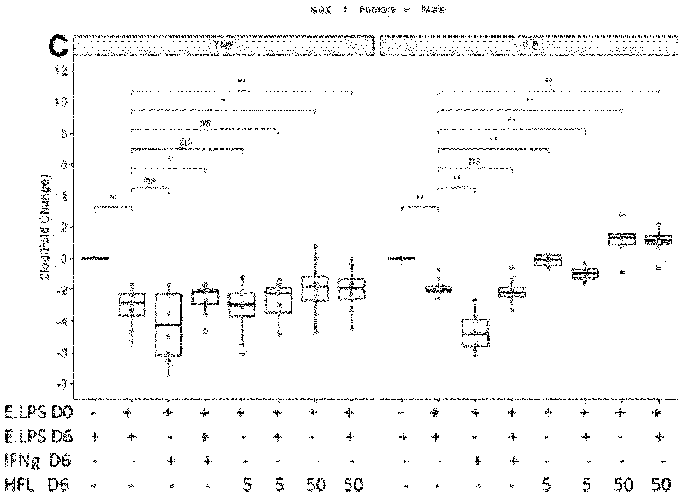

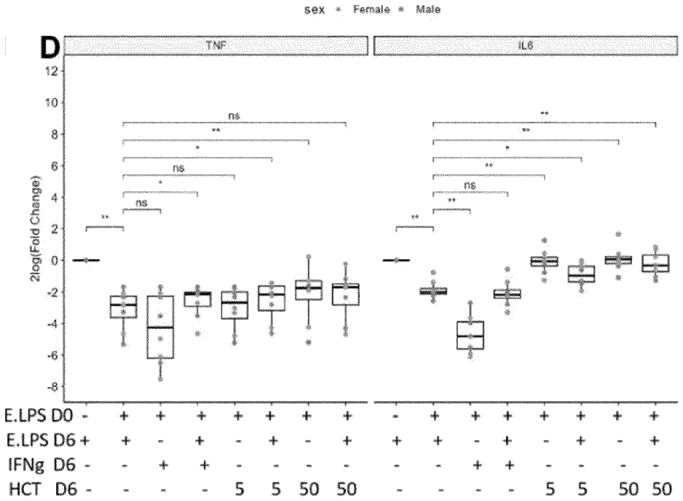

**Figure 3**: Figure 8 Cytokine production by naive and LPS-tolerant **monocytes incubated with different reversal agents in the presence and absence of LPS.** Tumor Necrosis Factor alpha (TNF) and interleukin- 6 (IL-6) production by naive and LPS-tolerant percoll monocytes incubated with different reversal agents in the presence and absence of LPS. On day 0 (D0), cells were incubated for 24-hours with RPMI (E.LPS D0 -) or *E.coli* LPS 1 ng/mL on day 0 (E.LPS D0 +) for 24 hours. On day 6, naive or LPS-tolerant monocytes were restimulated for 24 hours with the following reversal stimuli: Interferon-gamma 10 ng/mL (IFNg D6, panel **A-D**), recombinant filarial HSP70 full-length (WFL D6, panel **A**), recombinant filarial HSP70 C-terminal domain (WCT D6, panel **B**), recombinant human HSP70 full-length (HFL D6, panel **C**), recombinant human HSP70 C-terminal domain (HCT D6, panel **D**) in the presence and absence of *E.coli* LPS 10 ng/mL (E.LPS D6). Data are represented as box and whiskers showing the log2 fold change in cytokine response compared with naive monocytes stimulated with LPS on day 6. Concentrations depicted in the figures are in nM. Statistical testing was performed using Wilcoxon signed rank tests. * P < 0.05, ** P < 0.01.

### Examples

### Materials and methods

### Assess whether different forms of HSP70 reverse immunoparalysis

The potential of different forms of filarial (*Setaria digitata*) and human HSP70 in reversing tolerance in human monocytes was evaluated using an *in vitro* model for immunoparalysis as described in previous studies (Jansen et al. 2023). This model uses *E. coli* LPS (E.LPS) to induce endotoxin tolerance. PBMCs and monocytes from 15 healthy donors (8 males, 7 females) were isolated. Subsequently, 1.5×10⁵ monocytes were incubated with E.LPS (1 ng/mL) for 24 hours to induce tolerance, while cells of the negative control condition were incubated with RPMI⁺⁺⁺ only. Both conditions were supplemented with 10% HPS. Following this initial LPS exposure, cells were rested for 4 days in RPMI⁺⁺⁺ supplemented with 10% HSPS after which they were restimulated with various forms of HSP70 (at concentrations of 0.5, 5 and 50 nM) or IFN-y (10 ng/mL, positive control) in the presence or absence of *E.coli* LPS 10 ng/mL for 24 hours in an incubator. Supernatant was stored at -20 °C until cytokine measurements by ELISA.

The following HSP70 forms and variants were tested:
1) full length human HSP70 (HFL; sequence of GenBank: NP_005336.3)
2) C-terminal domain of human HSP70 (HCT; amino acids 388-641 of sequence of GenBank: NP_005336.3)
3) full length *Setaria digitata* HSP70 (WFL; sequence of GenBank: AAD13154.1)
4) C-terminal domain of *Setaria digitata* HSP70 (WCT; amino acids 388-645 of sequence of GenBank: AAD13154.1)

### ELISA

Cytokine levels of Interleukin (IL)-6, IL-16, IL-10, and tumor necrosis factor (TNF) were measured in supernatant with human duoset enzyme-linked immunosorbent assay (ELISA) kits according to manufacturer's instructions (R&D Systems, DY206, DY201, DY217B, DY210). In the case of incubation with stimuli over a period of 7 days, the cytokine levels of IL-17, IL-22, and interferon(IFN)-γ were measured in the supernatant using human duoset ELISA kits, following the manufacturer's instructions (R&D Systems, DY317, DY782, DY285B).

### Statistical analysis

All data were analyzed using R version 4.1.3. Data are presented as box and whisker plots or line graphs. Wilcoxon signed-rank tests were performed to compare ELISA cytokine levels, percentage cell death, and total luminescence (ROS assay) between different conditions. Significance was defined as P < 0.05. Olink proteomic data were analyzed using the 'OlinkAnalyze' package provided by Olink. Paired T tests for the unique proteins were performed to compare the relative protein levels between two conditions and the Benjamini-Hochberg method was used to correct for multiple testing. Protein expression was considered significantly different between two conditions if the adjusted p-value was < 0.05. Differential expression of proteins was visualized by volcano plots and the data underwent dimensional reduction through PCA analysis. Heatmaps were generated to visualize patterns and relationships between the different stimuli. Line plots, Boxplots, Heatmaps, volcano plots, and PCA plots were created using the packages 'ggplot2' (version 3.4.4), 'ggpubr' (version 0.6.0) and `stats' (version 4.1.3).

### Results

### Filarial and human HSP70 reverse immunoparalysis

To assess whether the different variants of HSP70 proteins reverse immunoparalysis, monocytes were initially stimulated with E.LPS for 24 hours and subsequently allowed to rest for 4 days before being restimulated with E.LPS for 24 hours in the presence of different forms of HSP70 or interferon-gamma (IFN-y), a cytokine which has been shown to reverse immunoparalysis. As expected, cells that were initially stimulated with E.LPS showed a significantly reduced TNF and IL-6 response upon E.LPS restimulation compared with cells that were not initially incubated with E.LPS (log2 fold change data depicted in Figure 7, absolute cytokine concentrations shown in Supplementary Figure 6). The log2 fold change describes the change of immune activity compared to the suppressed (immuneparalyzed) control. The change is expressed as log2 fold of the measured INF or IL-6 levels.

This endotoxin-tolerant phenotype, which resembles immunoparalysis, was reversed by IFN-y in terms of TNF, but not of IL-6 production (Figure 7 and Supplementary Figure 6).

Conversely, 50 nM of all forms of HSP70 proteins effectively reversed the tolerant phenotype for both cytokines, and for most forms, significant reversal was already observed at 5 nM (Figure 7 and Supplementary Figure 6). Depending on the dose, complete reversal of immunoparalysis was possible. Of note, in PBMCs obtained from female donors the reversal effects of the HSP70 proteins appeared stronger than in PBMCs obtained from males.

Next, we investigated if the HSP70 forms could reverse endotoxin tolerance without co-incubation with E.LPS on day 6. Of note, TNF responses in this set of experiments were very low, therefore the IL-6 results were focused on (log2 fold change data depicted in Figure 8, absolute cytokine concentrations shown in Supplementary Figure 7). The different HSP70 proteins were also capable to reverse endotoxin tolerance in the absence of E.LPS, an effect which was not observed for IFN-y.

### References

Bundschuh, D. S., Barsig, J., Hartung, T., Randow, F., Döcke, W. D., Volk, H. D., Wendel, A. (1997) Granulocyte-macrophage colony-stimulating factor and IFN-gamma restore the systemic TNF-alpha response to endotoxin in lipopolysaccharide-desensitized mice. J. Immunol. 158, 2862-2871.
Cajander et al. (Lancet Respir Med 2024, volume 12, issue 4, P305-322; doi: 10.1016/S2213-2600(23)00330-2).
Chen, J., Ivashkiv, L. B. (2010) IFN-g abrogates endotoxin tolerance by facilitating toll-like receptor-induced chromatin remodeling. Proc. Natl. Acad. Sci. USA 107, 19438-19443.
Döcke, W. D., Randow, F., Syrbe, U., Krausch, D., Asadullah, K., Reinke, P., Volk, H. D., Kox, W. (1997) Monocyte deactivation in septic patients: restoration by IFN-gamma treatment. Nat. Med. 3, 678-681.
Jansen A, Bruse N, Waalders N, Gerretsen J, Rijbroek D, Pickkers P, Kox M. Ex vivo and in vitro Monocyte Responses Do Not Reflect in vivo Immune Responses and Tolerance. J Innate Immun. 2023;15(1):174-87.
Leentjens, J., Kox, M., Koch, R. M., Preijers, F., Joosten, L. A., van der Hoeven, J. G., Netea, M. G., Pickkers, P. (2012) Reversal of immunoparalysis in humans in vivo: a double-blind, placebo-controlled, randomized pilot study. Am. J. Respir. Crit. Care Med. 186, 838-845.
Liu S, Luo W, Szatmary P, Zhang X, Lin JW, Chen L, Liu D, Sutton R, Xia Q, Jin T, Liu T, Huang W. Monocytic HLA-DR Expression in Immune Responses of Acute Pancreatitis and COVID-19. Int J Mol Sci. 2023 Feb 7;24(4):3246. doi: 10.3390/ijms24043246.
Miles RH, Paxton TP, Dries DJ, Gamelli RL (1994) Interferongamma increases mortality following cecal ligation and puncture. J. Trauma 36, 607-611.
Murphey, E. D., Herndon, D. N., Sherwood, E. R. (2004) Gamma interferon does not enhance clearance of Pseudomonas aeruginosa but does amplify a proinflammatory response in a murine model of postseptic immunosuppression. Infect. Immun. 72, 6892-6901.
Murphey, E. D., Sherwood, E. R. (2006) Bacterial clearance and mortality are not improved by a combination of IL-10 neutralization and IFNgamma administration in a murine model of post-CLP immunosuppression. Shock 26, 417-424.

## Claims

1. A compound comprising Heat Shock Protein 70 (Hsp70) or a therapeutically active part thereof for use in the treatment or prevention of immune suppression in an individual.

2. A compound comprising Heat Shock Protein 70 (Hsp70) or a therapeutically active part thereof for use in reversing immune suppression or inducing trained immunity in an individual.

3. The compound according to claim 1 or 2 wherein the immune suppression is immunoparalysis.

4. A compound comprising Heat Shock Protein 70 (Hsp70) or a therapeutically active part thereof for use in preventing or counteracting post-trauma infection, such as post-injury infection, secondary infection, and post-surgery infection or complications of such post-trauma infection in an individual.

5. The compound according to claim 4, wherein post-injury infection, secondary infection, infection following trauma or complications of such infection is prevented or counteracted by counteracting immunoparalysis.

6. The compound according to any one of the preceding claims wherein the individual is suffering from trauma.

7. The compound according to any one of the preceding claims wherein survival and/or quality of life is improved, the need for life support is avoided or the duration thereof reduced, and/or long term adverse health effects are prevented or reduced.

8. The compound according to any one of the preceding claims wherein the individual is suffering from bacterial infection, viral infection, critical illness, chronic psychological stress, sepsis, septic shock, multiple organ dysfunction syndrome (MODS), (severe) physical injury, stroke, such as acute ischemic stroke, spinal cord injury, liver ischemia-reperfusion injury, myocardial infarction, cystic fibrosis, acute coronary syndrome (ACS), pancreatitis, and/or cancer, and/or has undergone surgery, transplantation, anticancer therapy, chemotherapy, immunosuppressant therapy, long term hospitalisation and/or Intensive Care Unit (ICU) admission.

9. A compound comprising Heat Shock Protein 70 (Hsp70) or a therapeutically active part thereof for use in treating trauma and/or complications thereof, wherein the trauma is selected from the group consisting of critical illness, chronic psychological stress, (severe) physical injury, stroke, such as acute ischemic stroke, spinal cord injury, liver ischemia-reperfusion injury, myocardial infarction, cystic fibrosis, acute coronary syndrome (ACS), pancreatitis, and/or cancer, and/or results from surgery, transplantation, anticancer therapy, immunosuppressing therapy, chemotherapy, long term hospitalisation and/or Intensive Care Unit (ICU) admission.

10. A compound comprising Heat Shock Protein 70 (Hsp70) or a therapeutically active part thereof for use in improving the efficacy of anticancer therapy in an individual.

11. The compound according to any one of the preceding claims, wherein the individual is suffering from immunoparalysis.

12. The compound according to claim 8 or 9 wherein the surgery is a major surgical procedure, cardiopulmonary bypasses, coronary artery bypass grafting.

13. The compound according to claim 8, 9 or 10 wherein the anticancer therapy is chemotherapy, radiation therapy or immune therapy.

14. The compound according to any one of the preceding claims wherein the therapeutically active part comprises the C-terminal domain of Hsp70, preferably wherein the therapeutically active part of Hsp70 comprises amino acids 508-637, amino acids 508-641, amino acids 397-641 or amino acids 388-641 of human Hsp70 or the corresponding amino acids of Hsp70 of a non-human species.

15. The compound according to any one of the preceding claims wherein the Hsp70 is human Hsp70, *Setaria digitata* Hsp70, *Wuchereria bancrofti* Hsp70 or *Brugia malayi* Hsp70.
